(19) Europäisches Patentamt
European Patent Office
Office européen des brevets

(11) EP 3 294 451 B1

(12) **EUROPEAN PATENT SPECIFICATION**

(45) Date of publication and mention
of the grant of the patent:
**25.02.2026 Bulletin 2026/09**

(21) Application number: **16792316.8**

(22) Date of filing: **04.03.2016**

(51) International Patent Classification (IPC):
**B01J 23/00** (2006.01)    **B01J 23/63** (2006.01)
**B01J 23/652** (2006.01)    **B01J 35/70** (2024.01)
**C07C 5/27** (2006.01)    **C10G 25/05** (2006.01)

(52) Cooperative Patent Classification (CPC):
(C-Sets available)
**B01J 23/63; B01J 21/066; B01J 23/002;
B01J 23/005; B01J 23/42; B01J 23/6527;
B01J 23/83; B01J 23/8986; B01J 35/633;
B01J 35/70; B01J 35/733; B01J 37/0201;
B01J 37/031; B01J 37/033; C07C 5/27;**    (Cont.)

(86) International application number:
**PCT/IN2016/000061**

(87) International publication number:
**WO 2016/181407 (17.11.2016 Gazette 2016/46)**

(54) **ADDITIVE COMPOSITION FOR MIXED METAL OXIDE CATALYSTS AND ITS USE IN HYDROCARBON CONVERSION PROCESSES**

ADDITIVZUSAMMENSETZUNG FÜR MISCHMETALLOXIDKATALYSATOREN UND DEREN VERWENDUNG IN KOHLENWASSERSTOFFUMWANDLUNGSVERFAHREN

COMPOSITION D'ADDITIF POUR CATALYSEURS D'OXYDES MÉTALLIQUES MIXTES ET SON UTILISATION DANS DES PROCÉDÉS DE CONVERSION D'HYDROCARBURES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.05.2015 IN 1840MU2015**

(43) Date of publication of application:
**21.03.2018 Bulletin 2018/12**

(73) Proprietor: **Viridis Chemicals Private Limited**
**Mumbai, Maharashtra 400 021 (IN)**

(72) Inventor: **SAMPARA, Chaitanya**
**Mumbai**
**Maharashtra 400 021 (IN)**

(74) Representative: **Page White Farrer**
**Bedford House**
**21a John Street**
**London WC1N 2BF (GB)**

(56) References cited:
EP-A1- 0 532 024        EP-A1- 0 558 148
EP-A1- 1 002 579        EP-A1- 1 914 216
EP-B1- 2 100 663        US-A1- 2005 161 368
US-A1- 2014 219 878    US-B2- 9 040 762

(52) Cooperative Patent Classification (CPC): (Cont.)
**C10G 11/04; C10G 29/205; C10G 45/00;**
**C10G 45/60; C10G 47/04; C10G 50/00;**
B01J 2235/15; B01J 2523/00

C-Sets
B01J 2523/00, B01J 2523/24, B01J 2523/31,
B01J 2523/32, B01J 2523/36, B01J 2523/3712,
B01J 2523/48, B01J 2523/72, B01J 2523/821;
B01J 2523/00, B01J 2523/24, B01J 2523/31,
B01J 2523/32, B01J 2523/36, B01J 2523/3712,
B01J 2523/48, B01J 2523/72, B01J 2523/828;

B01J 2523/00, B01J 2523/24, B01J 2523/31,
B01J 2523/32, B01J 2523/3712, B01J 2523/3737,
B01J 2523/48, B01J 2523/72, B01J 2523/828;
B01J 2523/00, B01J 2523/24, B01J 2523/3712,
B01J 2523/3737, B01J 2523/72;
B01J 2523/00, B01J 2523/24, B01J 2523/3712,
B01J 2523/3737, B01J 2523/821;
B01J 2523/00, B01J 2523/24, B01J 2523/3712,
B01J 2523/3787, B01J 2523/72;
B01J 2523/00, B01J 2523/31, B01J 2523/48,
B01J 2523/72, B01J 2523/828, B01J 2523/842

**Description**

**FIELD OF THE INVENTION**

The present invention relates to an additive composition which when added to mixed metal oxide catalysts increases the performance of the mixed metal oxide catalyst and requires lower usage of group VIII metals *viz*. Platinum group metals which are commonly used for increasing the life of the catalyst. The present invention also relates to a process for preparing the afore-mentioned additive composition. The present invention further relates to hydrocarbon conversion processes carried out using the mixed metal oxide catalysts which comprises the additive composition.

**BACKGROUND OF THE INVENTION**

Chlorine doped alumina catalysts and Zeolites have been popular catalysts for several hydrocarbon reactions used in petroleum processes, specifically isomerization reactions. While Cl⁻/$Al_2O_3$ catalysts exhibit excellent activity and selectivity towards hydrocarbon reactions, they are highly sensitive to feed contaminants such as water ($H_2O$), organic sulfur and organic nitrogen and hence require highly capital intensive feed pretreatment procedures for their usage. They further produce harmful hydrochloric acid downstream which needs to be treated adding to the process costs. Zeolites on the other hand do not require pre- or post-treatment but are significantly lower in terms of their activity. A tradeoff thus exists between the two catalysts and refiners are often left to choose between both the popular options either trading off in terms of performance or costs.

An optimal hydrocarbon conversion catalyst specifically for isomerization reactions possesses the following traits. It is important to satisfy all three criteria described below for superior performance.

a. Low temperature operation: Isomerization reaction is an equilibrium reaction and the forward reaction which is desired for maximum product yield is facilitated at low temperatures (<135°C).

b. High tolerance to feed contaminants and efficient hydrogen splitting: Both these attributes typically are obtained from the metallic sites on the doped support. While the former attribute shields the catalyst towards feed contaminants such as sulfur and nitrogen, the latter attribute facilitates efficient use of hydrogen on the surface thus minimizing its usage in the feed and also suppressing coke formation on the surface.

c. Optimal pore structure of the support: This is required to maximize the selectivity of the desired products.

Hino et al. (1980) in their seminal paper - "Synthesis of Solid superacid catalyst with acid strength of H0 < -16.04", J.C.S. Chem. Comm., 851 - 852, 573, 1980 explained the usage of anion modified Group III and Group IV oxides such as $WO_3$/$ZrO_2$ and $SO_4^{2-}$/$ZrO_2$ catalysts as strong acids which exhibit promising performance in hydrocarbon conversion processes such as isomerization. While these catalysts show moderate performance tradeoff in comparison to the Cl⁻/$Al_2O_3$ catalysts, they do not need the feed pretreatment and thus the tradeoff between costs and performance is to a lower extent for the refiner in comparison to the Zeolite catalysts. While several patents and papers have been published improving the design of the originally proposed catalyst by Hino *et al.* prolonged catalyst stability was not achieved which is essential for commercial petroleum applications. This prior art describes the usage of sulfated zirconia catalysts which at most satisfy two of the above attributes thus leading to non-optimal performance in hydrocarbon conversion processes such as isomerization.

More recently substantial research has been conducted to improve the stability and longevity of these anion modified acid catalysts and several patents have been filed exhibiting their advanced stability for petroleum processes such as isomerization, alkylation and transalkylation - US Patent No. 6107235 and 6080904 etc.

Further, the use of several dopant systems is reported to increase the stability of these catalyst compositions, for example, Russian patent document 2191627 discloses a method for the synthesis of sulfated zirconia catalyst loaded with noble metals such as platinum, palladium, ruthenium, osmium, iridium and the like on an zirconia support containing up to 20 % of active components of silicon, titanium oxide, magnesium and alumina. The catalyst is further loaded with Nickel, Titanium, Germanium, Manganese, Cobalt, Bismuth, Iron, Vanadium, Cobalt, Zirconium and mixtures thereof. The catalyst as disclosed in the aforementioned patent is used for the isomerization reaction; however, the catalyst demonstrates very poor stability. The concentration of 2, 2-Dimethyl butane (2, 2-DMB) a key performance metric in the product stream decreases after 200 hours operation from 28% to 14%. This catalyst formulation also requires higher quantities of Platinum group (Group VIII) metals to maintain performance over extended periods of operation.

US Patent No. 3032599 disclosed one of the first usages of sulfated zirconia to isomerization and alkylation of hydrocarbons. The catalysts showed high initial activity but not prolonged performance due to low surface area. Furthermore, these powdery catalysts are for the most part unusable in industrial reactors due to pressure drop implications.

US Patent No. 3132110 discloses the use of sulfated zirconia catalyst, pure or preferably combined with alumina.

The catalysts obtained herein are indeed excellent in terms of initial activity but do not show prolonged performance. Further, they need very high operating temperatures of over 370°C which results in poor product yields. Also, these catalysts undergo accelerated deactivation due to coke deposition on the surface during operation.

US Patent No. 6448198 discloses the synthesis of high surface area and mesopore zirconia structures which when sulfated gives superior performance with isomerization reactions. However, these catalysts suffer with significant inhibition from feed contaminants such as organic sulfur and nitrogen. Their performance in terms of peak yield of the desired product 2, 2-DMB degrades from 27% to 12% in the presence of 5ppm of either sulfur or nitrogen in the feed stream.

European patent application No. 1002579 discloses a layered catalyst having zirconium core component followed by Mn, Fe or Ni shell and a top layer consisting of noble metals. The catalyst was used for the isomerization reaction. However, the catalyst shows degradation in overall performance after 200 hours of operation. The concentration of 2, 2-DMB, a key performance product decreases from 28% to 20%.

Another Russian patent document 2171713 discloses a process for the preparation of a catalyst with 0.2 to 1% platinum or Palladium, 0.05 to 2.5 % chlorine and 0.5 to 10 % sulfate which are deposited on a mixture of aluminum and zirconium oxide. The major disadvantage of this catalyst is low stability for isomerization reaction and higher use of reactant hydrogen. A hydrogen to oil ratio of 5 to 10 is required to maintain stability of such a catalyst over extended operating periods.

US Patent No. 7015175 discloses a method for the synthesis of sulfated zirconium catalyst doped with high concentration (of at least 3 %) of Lanthanide series elements. The performance of such a catalyst degrades substantially in the presence of organic sulfur or nitrogen based contaminants which are typically seen in the petroleum feed stream. The yield of 2, 2-DMB reduces from 28% to 14% with 5ppm of sulfur in the feed stream. Also, the operating temperature as described in the patent is substantially high (~ 170°C) for optimal operation. Isomerization is an equilibrium reaction and high conversion is facilitated at low temperatures (~ 130°C).

Further, US 2014/0219878 A1 (published 7 August 2014) is directed to mixed phase catalysts. US 2005/0161368 A1 (published 28 July 2005) is directed to high-activity isomerization catalyst and process. EP 1002579 A1 (published 24 May 2000) is directed to a sulfated layered catalyst for use in a paraffin isomerization process. US 9040762 B2 (published 26 May 2015) is directed to catalysts for petrochemical catalysis. EP 0532024 A1 (published 17 March 1993) is directed to a catalyst for catalytic reduction of nitrogen oxide. EP 0558148 A1 is directed to a process for the dehydrogenation of organic compounds.

Thus in order to overcome the limitations of the prior arts described above, the present invention provides a highly efficient additive composition which can be directly added to the mixed metal oxide catalysts. The additive composition increases the performance of the catalyst, favors low temperature operations, increases tolerance to feed contaminants and decelerates the catalyst growth for high product selectivity.

## SUMMARY OF THE INVENTION

In one aspect the present invention provides an additive composition according to claim 1.

In another aspect the present invention provides a process for preparing the afore-mentioned additive composition according to claim 3.

Another aspect of the present invention provides a catalyst comprising the afore-mentioned additive composition according to claim 9.

Yet another aspect of the present invention provides a process for converting hydrocarbons according to claim 14.

Further scope and applicability of the present invention will become apparent from the detailed description given hereinafter. It is to be understood that both the foregoing general description and the following detailed description and specific examples are exemplary and explanatory only and are not restrictive of the invention, which is defined by the claims.

## BRIEF DESCRIPTION OF THE DRAWINGS:

The following detailed description of the invention will be better understood when read in conjunction with the appended drawings. For the purpose of assisting in the explanation of the invention, there are shown in the drawings embodiments which are presently preferred and considered illustrative. It should be understood, however, that the invention is not limited to the precise arrangements and instrumentalities shown therein.

**Figure 1:** X-ray diffraction pattern of additive composition for catalyst Z1 of the present invention which shows the onset of the crystal formation.

**Figure 2:** X-ray diffraction pattern of additive composition for catalyst Z2 of the present invention which shows the onset of the crystal formation.

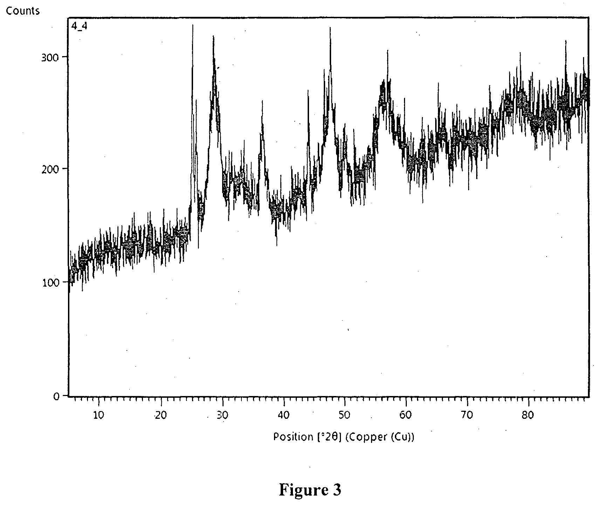

**Figure 3:** X-ray diffraction pattern of additive composition for catalyst Z3 of the present invention which shows the onset of the crystal formation.

## DETAILED DESCRIPTION OF THE INVENTION

For the purposes of the following detailed description, it is to be understood that the invention may assume various alternative variations and step sequences, except where expressly specified to the contrary. Moreover, other than in any operating examples, or where otherwise indicated, all numbers expressing, for example, quantities of ingredients used in the specification are to be understood as being modified in all instances by the term "about". It is noted that, unless otherwise stated, all percentages given in this specification and appended claims refer to percentages by weight of the total composition.

Thus, before describing the present invention in detail, it is to be understood that this invention is not limited to particularly exemplified systems or process parameters that may of course, vary. It is also to be understood that the terminology used herein is for the purpose of describing particular embodiments of the invention only, and is not intended to limit the scope of the invention in any manner.

The use of examples anywhere in this specification including examples of any terms discussed herein is illustrative only, and in no way limits the scope and meaning of the invention or of any exemplified term. Likewise, the invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions will control.

It must be noted that, as used in this specification and the appended claims, the singular forms "a," "an" and "the" include plural referents unless the content clearly dictates otherwise.

The terms "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, the terms "comprising" "including," "having," "containing," "involving," and the like are to be understood to be open-ended, i.e., to mean including but not limited to.

Described herein is an additive composition comprising mixed metal oxide having the general formula:

$$A_xB_yC_{(1-y)}D_zO_m$$

wherein:

A is one or more metal elements selected from the group consisting of Group IIA of the periodic table;
B, C is one or more metal elements selected from the lanthanide group series of the periodic table or Yttrium;
D is one or more metal elements selected from the group consisting of Manganese, Cobalt, Copper, Nickel or Ruthenium;
x is a number defined by $0.5 < x < 4$
y is a number defined by $0 <= y <= 1$
z is a number defined by $2 < z < 6$
m is a number which renders the catalyst substantially neutral.

In one example of the description Element A is selected from the group consisting of Strontium, Magnesium, Barium or mixtures thereof. Elements B and C are selected from the group consisting of Cerium, Samarium, Ytterbium, Lanthanum, Neodymium, Yttrium or mixtures thereof. However, according to the invention element A is Strontium, B is Cerium or Yttrium, C is Samarium or Ytterbium and D is Manganese or Ruthenium.

In an embodiment of the present invention, the mole percent of element A is in the range of $0.1 < A < 0.25$; mole percent of elements B, C are in the range of $0.05 < B, C < 0.2$; mole percent of element D is in the range of $0.1 < D < 0.35$; and the valence charge of oxygen is such that the overall charge of the molecule is neutral.

In the most preferred embodiment of the invention, the additive composition has the general formula:

$$Sr_xB_yC_{(1-y)}D_zO_m$$

wherein:

B is Cerium or Yttrium;
C is Samarium or Ytterbium;
D is Manganese or Ruthenium;
x is a number defined by $0.5 < x < 4$
y is a number defined by $0 <= y <= 1$
z is a number defined by $2 < z < 6$
m is a number which renders the overall charge substantially neutral.

Further provided is a process for preparing the afore-mentioned additive composition comprising:

a. dissolving water soluble salts of A, B, C and D to obtain the general formula of the afore-mentioned additive composition;
b. adding a precipitating agent to the resultant mixture of step (a);
c. adjusting the pH of the solution of step (b) to about 9 - 12;
d. filtering and washing the resulting precipitant of step (c);
e. calcining the precipitant of step (d) at 300°C to 600°C for 1 hour to 6 hours to obtain the additive composition.

The process according to the invention is according to claim 3. In an embodiment the additive composition may be prepared by co-precipitation method or sequential precipitation method wherein suitable amounts of metal salt precursors such as but not limited to nitrate salts or acetate salts or chloride salts or sulfate salts are dissolved in water. The resultant mixture of metal cations is then precipitated using a precipitating agent. The precipitating agent may also act as a surfactant to create the desired crystal structure. Suitable precipitating agent may be selected from the group consisting of sodium hydroxide, sodium carbonate, oxalic acid, sodium oxalate, ammonium oxalate or mixtures thereof and may be added in an amount so as to obtain a final pH value of at least 9 or above.

The pH of solution of step (b) is adjusted between 9 - 12 using tetramethylammonium hydroxide (TMAOH). Additional TMAOH may be added to reestablish the pH of the solution. The resultant metal precipitant is filtered and washed using deionized water for three to four times. Finally, the metal precipitant is dried, processed and calcined at 500°C for 4 hours.

The afore-mentioned process of the invention comprises adding an oxidizing agent to the mixture of step (a). The process may optionally comprise adding a surfactant to the mixture of step (a). Said oxidizing agent may be selected from the group consisting of hydrogen peroxide, chlorate, perchlorate, hypochlorite, nitric acid, sulfuric acid, potassium permanganate or mixtures thereof and may be added in an amount of 100% in excess of the moles of element D used in the additive composition. Said surfactant may be selected from the group consisting of polyvinyl alcohol (PVA), Triton X-100, Pluronic F127, Polyethylene glycol (PEG), sodium salt of polyacrylic acid (Na-PAA) or mixtures thereof and may be added in an amount of 50% in excess of the moles of element A used in the additive composition.

The additive composition may be partially calcined to start the formation of the desired crystals which when mixed with the mixed metal oxide catalyst such as the sulfated alumina zirconia catalyst will allow optimal crystal formation during final calcination procedure. In another embodiment of the present invention, the additive may not be fully calcined wherein X-ray diffraction does not reveal fully crystalline structures but only reveals partially crystalline and partially amorphous phases.

In another embodiment of the present invention, the additive composition may be prepared using the citric acid method. Said method includes dissolving suitable amounts of the different metal salts in water with 10% excess of molar amounts of citric acid. Optionally, all or a portion of the citric acid may be substituted with Ethylene diamine tetraacetic acid (EDTA) or a mixture of Polyvinyl alcohol (PVA) and sucrose. The mixture is stirred and heated until a viscous gel forms. The viscous gel is dried, processed, and calcined at 500°C for 4 hours.

A mixed metal oxide catalyst is provided comprising the afore-mentioned additive composition. According to the invention a catalyst is provided comprising the additive composition of the invention and a mixed metal oxide catalyst, optionally wherein the metal of the mixed metal oxide catalyst is selected from the group consisting of Group III and Group IV of the periodic table.

In an embodiment of the present invention, the mixed metal oxide catalyst is selected from the group consisting of a sulfated or a tungsated metal oxide of Zirconium or Aluminum or mixtures thereof.

The afore-mentioned mixed metal oxide catalyst may further comprise a Group VIII metal selected from the group consisting of Platinum, Palladium, Rhenium, Rhodium, Iridium, Ruthenium, Gold or mixtures thereof. In an embodiment of the present invention, about 0.1% to 5% of Group VIII metal may be present in the mixed metal oxide catalyst. Said Group VIII metal in small amounts may be added to the final mixed metal oxide catalyst by usual impregnation techniques known to those skilled in the art. One of the specific advantages of the additive composition of the present invention is to provide the optimal charge density on the surface catalyst to enable further ion doping. For example, most hydrocarbon processes require small quantities of Platinum group metals (PGM) to be further added to the final catalyst recipe to enhance long term performance. PGM is typically added to the catalyst by dry impregnation method which while cheaper does not give optimal control on the location of PGM on the surface of the catalyst leading to non-optimal performance. The additive composition described in the present invention when added to the catalyst will provide the interionic repulsive forces thus increasing the dispersion (or surface area) of the PGM metals on the surface, thus dramatically improving the PGM surface area. Larger PGM surface area results directly in increasing catalyst longevity and reactivity. Further, the additive also increases the metal support interactive forces thus decreasing the stronger binding forces of the PGM metals towards undesirable feed contaminants such as sulfur and nitrogen.

In an exemplary embodiment of the present invention, the additive composition may be directly added to sulfated zirconia catalysts as described in the open literatures titled 1) "Effect of isopropanol aging of Zr(OH)4 on n-hexane isomerization over Pt-SO42-/Al2O3-ZrO2, Catalysis Today, Oct 2009, 148 (1-2), p 70-74, 2) "Catalytic performance of Re/Ga2O3/WO3/ZrO2 catalyst for n-Hexane isomerization", Chi.J. of. Catal. Sept 2009, 30(9), p 859-863).

The additive composition can be directly added to mixed metal oxide catalysts in varying ratios to the sulfated zirconia catalyst such as $Pt\text{-}SO_4{}^{2-}/Al_2O_3/ZrO_2$ catalysts to obtain the desired results.

In an embodiment of the present invention, the additive composition is added to the mixed metal oxide catalyst prior to calcination and about 0.5 to 25 weight percent of the additive composition is added to the mixed metal oxide catalyst. In order for the final catalyst to be active, the sulfated mixture of $Al_2O_3$ and $ZrO_2$ in their hydroxide form should be intimately mixed with the additive composition in ratios of 2 to 25% so as to obtain maximum benefit.

The additive composition of the present invention may be synthesized separately from the mixed metal oxide catalyst (such as sulfated alumina zirconia catalyst). The synthesis of the sulfated alumina zirconia catalyst along with some transition metal additive may be done through the processes as described in the open literature titled 1) "Effect of isopropanol aging of Zr(OH)4 on n-hexane isomerization over Pt-SO42-/Al2O3-ZrO2, Catalysis Today, Oct 2009, 148 (1-2), p 70-74, and 2) "Catalytic performance of Re/Ga2O3/WO3/ZrO2 catalyst for n-Hexane isomerization", Chi.J. of. Catal., Sept 2009, 30(9), p 859-863. High surface area $Zr(OH)_4$ and pseudo boehmite may be mulled together and extruded using sulfate ion additive such as sulfuric acid or ammonium sulfate followed by drying at 120°C for several hours. In addition a transition metal ion may be added to increase the hydrogenation ability of the catalyst. In an embodiment of the present invention, the additive composition synthesized will have at least two phases namely a spinel phase and a mullite phase or a fluorite phase oxide when characterized with Transmission Electron Microscopy (TEM) or Scanning Transmission Electron Microscopy (STEM)/Energy-dispersive X-ray Analyser (EDX) techniques. In another embodiment, the spinel phase and/or the fluorite phase and/or the mullite phase can include one or more metals.

In a further embodiment of the present invention, the mixed phase catalyst additive composition may include a composite having two or more constituent materials with different physical or chemical properties which remain separate and distinct at the macroscopic or microscopic scale within the finished structure. For example, two particles with different compositions remain separate in TEM ("Transmission Electron Microscopy") while they do have common interface.

The present description provides a process for converting hydrocarbons by contacting a feed with the mixed metal oxide catalyst having the additive composition.

Said hydrocarbon conversion process may be selected from the group consisting of aromatic alkylation, isoparaffin alkylation, isomerization, polymerization, reforming, hydrogenation, transalkylation or dealkylation, preferably, isomerization, alkylation or transalkylation.

The additive composition described herein enhances the performance of mixed metal oxide catalysts towards hydrocarbon processing reactions such as but not limited to isomerization. The additive composition of the present invention specifically

(i) Creates optimal strength acid sites on the surface to facilitate low temperature operation of the catalyst, thus increasing product conversion.
(ii) Increases metal support interaction to minimize the effect of feed contaminants.
(iii) Aids in generating effective surface hydrogen by splitting gas phase hydrogen thus minimizing coke formation on the surface.
(iv) Decelerates the catalyst growth during calcination phase of the catalyst synthesis to maximize the mesopores required for high product selectivity.

The following examples are provided to better illustrate the claimed invention and are not to be interpreted in any way as limiting the scope of the invention. The specific compositions, materials, and methods described below are not intended to limit the invention, but merely to illustrate specific embodiments falling within the scope of the invention.

**Examples**

**Comparative Example 1: Catalyst A**

Catalyst A was prepared using the well-established sulfated alumina zirconia synthesis route as known to those skilled in the art as shown in US Pat No. 6,448,198 B1. The corresponding references for the catalyst design have been provided for reference. Briefly, high surface area Zirconium hydroxide ($Zr(OH)_4$) and pseudo-boehmite were co-mulled together to form an intimate mixture. They were then extruded into 1mm diameter and 2mm length extrudes. The extrudes were then exposed to 1M sulfuric acid (15 ml/gm of extrudes) and subsequently vacuum filtered to remove the excess sulfate ions. The extrudes were then calcined at 600°C for 3 hours to form crystalline sulfated alumina zirconia catalyst. The catalyst had 0.24 ml/g pore volume at the end of the calcination. Platinum with 0.3 wt% was then incorporated into the catalyst by wet impregnation technique as known to those skilled in the art. This catalyst was designated catalyst A.

7 gm of catalyst A was loaded in a fixed bed reactor where it was calcined in air at 500°C for 2 hours and subsequently reduced in Hydrogen for 1 hour. A reaction mixture containing the following composition was then flown on the catalyst at 30 bar and 135°C for 200 hours. The result of the product sample drawn at 50 and 200 hours respectively is shown in Table 2.

Table 1: Feed composition for fixed bed reactor testing of the catalyst compositions for activity towards hydrocarbon isomerization

| Reactant | Concentration |
|---|---|
| n-Pentane | ~ 32% |
| n-Hexane | ~ 55% |
| CycloHexane | ~ 8% |
| Benzene | ~ 5% |
| 1-Propanethiol | 7 ppm |
| Water | 10 ppm |
| Pressure of operation | 30 bar |
| Temperature of operation | 135°C |

**Comparative Example 2: Catalyst B**

Catalyst B was prepared using the popular metal doped sulfated zirconia catalyst synthesis route as known to those skilled in the art and also described in the patent, EP0532153B1. Briefly, high surface area zirconium hydroxide was prepared by rapidly mixing zirconium carbonate and ammonia solutions. After washing and sieving to desired size, Iron (Fe) and Manganese (Mn) nitrate solutions were added to the support material via incipient wetness impregnation method. The catalyst was subsequently dried and calcined at 725°C for one hour. 0.3% Platinum was incorporated into the catalyst via incipient wetness impregnation method.

The testing procedure described in example 1 was used to evaluate catalyst B's performance. The results of which are detailed in Table 2.

**Example 3: Catalyst Z**

Base catalyst was synthesized using the method described in the open literature (reference provided in the previous sections). Briefly, high surface zirconium hydroxide and pseudo boehmite were co-mulled together and impregnated with 1N $H_2SO_4$ by using 15ml/gm of the support material. Excess acid was drained and the sulfated support was dried at 120°C for several hours. Gallium was then introduced into the support by adding its corresponding nitrate salt to achieve 3% loading by incipient wetness technique. The mixture was then dried and is labeled **Catalyst Z.**

**Additive Composition 1 - Catalyst Z1**

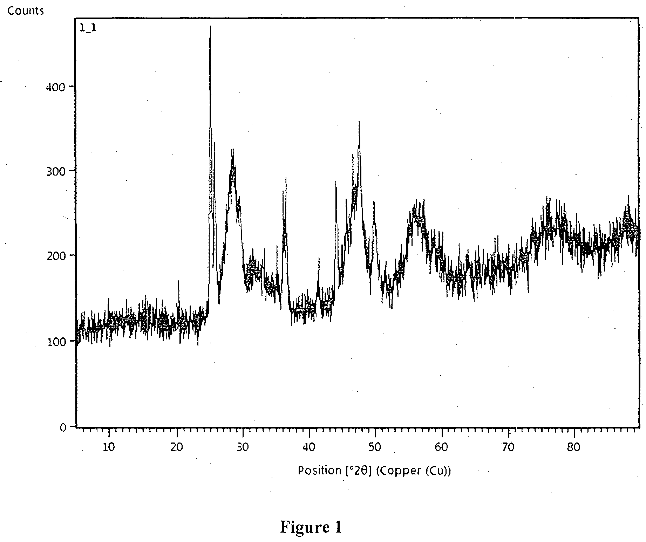

Additive for catalyst Z1 was first prepared by mixing nitrate salts of Strontium, Cerium, Samarium and Manganese. 25.5 gms of $Mn(NO_3)_2$ 50% solution, 5 gms of $Sr(NO_3)_2$, 9.3 gms of $Ce(NO_3)_3.6H_2O$ 1.1 gms of $Sm(NO_3)_3.6H_2O$ were mixed together in desired amount of water. 13.4 gms of 35% $H_2O_2$ was added to the mixture while stirring. 2.4 gms of Oxalic acid was then added to the mixture. Finally 1 gm of Pluronic (F127) and 1 gm of PEG were added to final mixture and stirred for 1 hour. The pH of the final solution was brought to 10.5 using TMAOH. The product was filtered, washed and calcined at 500°C. The XRD profile of the product is shown in figure 1. The molecular formula of the additive Z1 thus formed is $SrCe_{0.9}Sm_{0.1}Mn_3O_m$ where m is defined to render the overall charge neutral.

The additive thus produced is added to catalyst Z shown in example 1 and extruded into 1mm diameter and 2mm length extrudes. The final catalyst formulation was calcined at 700°C for 3 hours. 0.3 wt% Platinum was added to the calcined extrudes by incipient wetness impregnation method. The catalyst is labeled Z1 and the isomerization test results with the feed shown in comparative example 1 are shown in Table 2.

**Additive Composition 2 - Catalyst Z2**

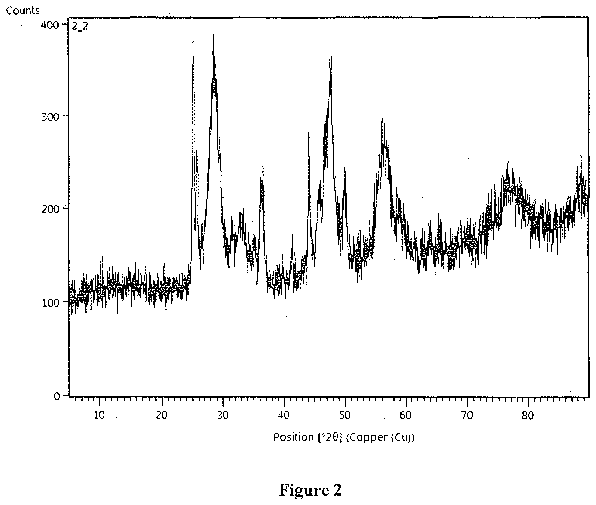

Additive for catalyst Z2 was first prepared by mixing nitrate salts of Strontium, Cerium, Ytterbium and Manganese. 25.5 gms of $Mn(NO_3)_2$ 50% solution, 5 gms of $Sr(NO_3)_2$, 9.3 gms of $Ce(NO_3)_3.6H_2O$ 1.1 gms of $Yb(NO_3)_3.6H_2O$ were mixed together in desired amount of water. 13.4 gms of 35% $H_2O_2$ was added to the mixture while stirring. 2.4 gms of Oxalic acid was then added to the mixture. Finally 1 gm of Pluronic (F127) and 1 gm of PEG were added to final mixture and stirred for 1 hour. The pH of the final solution was brought to 10.5 using TMAOH. The product was filtered, washed and calcined at 500°C. The XRD profile of the product is shown in figure 2. The molecular formula of the additive Z2 thus formed is $SrCe_{0.9}Yb_{0.1}Mn_5O_m$ where m is defined to render the overall charge neutral.

The additive thus produced is added to catalyst Z shown in example 1 and extruded into 1mm diameter and 2mm length extrudes. The final catalyst formulation was calcined at 700°C for 3 hours. 0.3 wt% Platinum was added to the calcined extrudes by incipient wetness impregnation method. The catalyst is labeled Z2 and the isomerization test results with the feed shown in comparative example 1 is shown in Table 2.

**Additive Composition 3 - Catalyst Z3**

Additive for catalyst Z3 was first prepared by mixing nitrate salts of Strontium, Cerium, Ytterbium and Ruthenium. 17 gms of $Ru(NO_3)_2$, 5 gms of $Sr(NO_3)_2$, 9.3 gms of $Ce(NO_3)_3.6H_2O$ 1.1 gms of $Yb(NO_3)_3.6H_2O$ were mixed together in desired amount of water. 13.4 gms of 35% $H_2O_2$ was added to the mixture while stirring. 2.4 gms of Oxalic acid was then added to the mixture. Finally 1 gm of Pluronic (F127) and 1 gm of PEG were added to final mixture and stirred for 1 hour. The pH of the final solution was brought to 10.5 using TMAOH. The product was filtered, washed and calcined at 500°C. The XRD profile of the product is shown in figure 2. The molecular formula of additive thus formed is $SrCe_{0.9}Yb_{0.1}Ru_2O_m$ where m is defined to render the overall charge neutral.

The additive thus produced is added to catalyst Z shown in example 1 and extruded into 1mm diameter and 2mm length extrudes. The final catalyst formulation was calcined at 700°C for 3 hours. 0.3 wt% Platinum was added to the calcined extrudes by incipient wetness impregnation method. The catalyst is labeled Z2 and the isomerization test result with the feed shown in comparative example 1 is shown in Table 2.

Table 2: Performance results for the catalysts (Z1, Z2 and Z3) prepared as per the invention in comparison to those known to prior art (A and B) shown in comparative examples

| Catalyst | Temperature of reaction | Pressure of reaction | $\dfrac{2,2-DMB}{n-Hex}$ 20 hour operation | $\dfrac{2,2-DMB}{n-Hex}$ 200 hour operation |
|---|---|---|---|---|
| A | 135°C | 30 bar | 23% | 12.0% |
| B | 135°C | 30 bar | 24% | 11.4% |
| Z1 | 135°C | 30 bar | 34.5% | 34.4% |
| Z2 | 135°C | 30 bar | 33% | 34.2% |
| Z3 | 135°C | 30 bar | 31% | 30.5% |

The catalysts prepared with the invention described herein, namely catalysts Z1, Z2 and Z3 exhibit excellent performance characteristics in terms of product conversion, low temperature operation and high tolerance to feed contaminants.

**Claims**

**1.** An additive composition comprising mixed metal oxide having the general formula:

$$A_xB_yC_{(1-y)}D_zO_m$$

wherein:

A is Strontium;
B is Cerium or Yttrium;
C is Samarium or Ytterbium;
D is Manganese or Ruthenium;
x is a number defined by $0.5 < x < 4$
y is a number defined by $0 <= y <= l$
z is a number defined by $2 < z < 6$
m is a number which renders the overall charge substantially neutral.

2. The additive composition as claimed in claim 1, wherein the mole percent of element A is in the range of $0.1 < A < 0.25$; the mole percent of element B, C are in the range of $0.05 < B, C < 0.2$; the mole percent of element D is in the range of $0.1 < D < 0.35$; and the valence charge of oxygen is such that the overall charge of the molecule is neutral.

3. A process for preparing the additive composition as claimed in claim 1 comprising:

a. dissolving water soluble salts of A, B, C and D to obtain the general formula as claimed in claim 1;
b. adding a precipitating agent to the resultant mixture of step (a);
c. adjusting the pH of the solution of step (b) to about 9 - 12;
d. filtering and washing the resulting precipitant of step (c);
e. calcining the precipitant of step (d) at 300°C to 600°C for 1 hour to 6 hours to obtain the additive composition,

wherein the process comprises adding an oxidizing agent to the mixture of step (a), optionally wherein the oxidizing agent is: (a) selected from the group consisting of hydrogen peroxide, chlorate, perchlorate, hypochlorite, nitric acid, sulfuric acid, potassium permanganate or mixtures thereof; and/or (b) added in an amount of 100% in excess of the moles of element D in the additive composition.

4. The process as claimed in claim 3, wherein the salts of A, B, C and D are selected from the group consisting of nitrate, acetate, chloride or sulfate.

5. The process as claimed in claim 3 or claim 4, wherein the process comprises adding a surfactant to the mixture of step (a), optionally wherein said surfactant is: (a) selected from the group consisting of polyvinyl alcohol, 4-(1,1,3,3, Tetramethylbutyl)phenyl-polyethylene glycol, poloxamer 407, Polyethylene glycol, sodium salt of polyacrylic acid or mixtures thereof; and/or (b) added in an amount of 50% in excess of moles of element A present in the additive composition.

6. The process as claimed in any of claims 3 to 5, wherein the precipitating agent is selected from the group consisting of sodium hydroxide, sodium carbonate, oxalic acid, sodium oxalate, ammonium oxalate or mixtures thereof and added in an amount to obtain a final pH value of at least 9.

7. The process as claimed in claim 3, wherein the pH of solution of step (b) is adjusted using tetramethylammonium hydroxide.

8. The process as claimed in any of claims 3 to 7, wherein the precipitant of step (d) is calcined at 500°C for 4 hours.

9. A catalyst comprising the additive composition as claimed in claim 1 or claim 2, and a mixed metal oxide catalyst, optionally wherein the metal of the mixed metal oxide catalyst is selected from the group consisting of Group III and Group IV of the periodic table.

10. The catalyst as claimed in claim 9, wherein the mixed metal oxide catalyst is selected from the group consisting of a sulfated or a tungsated metal oxide of Zirconium or Aluminum or mixtures thereof.

11. The catalyst as claimed in claim 9 or claim 10, wherein said mixed metal oxide catalyst further comprises a Group VIII metal selected from the group consisting of Platinum, Palladium, Rhenium, Rhodium, Iridium, Ruthenium, Gold or mixtures thereof.

12. The catalyst as claimed in any of claims 9 to 11, wherein the additive composition has a fluorite phase and/or a spinel phase and/ or a mullite phase when characterized with Transmission Electron Microscopy or Scanning Transmission Electron Microscopy / Energy-dispersive X-ray Analyser techniques.

13. A method of making a catalyst comprising the process of any of claims 3 to 8, wherein the additive composition is added to a mixed metal oxide catalyst, optionally wherein about 0.5 to 25 weight percent of the additive composition is added to the mixed metal oxide catalyst, and further optionally wherein the method comprises a step of calcination after the additive composition is added to the mixed metal oxide catalyst.

14. A process for converting hydrocarbons by contacting a feed with the catalyst as claimed in any of claims 9 to 12, wherein the hydrocarbon conversion process is selected from the group consisting of isomerization, alkylation or transalkylation.

**Patentansprüche**

1. Additivzusammensetzung, umfassend ein Mischmetalloxid mit der allgemeinen Formel:

$$A_xB_yC(_{1-y})D_zO_m$$

wobei

A Strontium ist;
B Cer oder Yttrium ist;
C Samarium oder Ytterbium ist;
D Mangan oder Ruthenium ist;
x eine Zahl ist, die durch $0,5 < x < 4$ definiert ist
y eine Zahl ist, die durch $0 <= y <= 1$ definiert ist
z eine Zahl ist, die durch $2 < z < 6$ definiert ist
m eine Zahl ist, die die Gesamtladung im Wesentlichen neutral macht.

2. Additivzusammensetzung nach Anspruch 1, wobei die Molprozent von Element A im Bereich von $0,1 < A < 0,25$ liegen; die Molprozent von Element B, C im Bereich von $0,05 < B, C < 0,2$ liegen; die Molprozent von Element D im Bereich von $0,1 < D < 0,35$ liegen; und die Valenzladung von Sauerstoff derart ist, dass die Gesamtladung des Moleküls neutral ist.

3. Verfahren zur Herstellung der Additivzusammensetzung nach Anspruch 1, umfassend:

a. Lösen von wasserlöslichen Salzen von A, B, C und D, um die allgemeine Formel nach Anspruch 1 zu erhalten;
b. Zugeben eines Fällungsmittels zu dem resultierenden Gemisch von Schritt (a);
c. Justieren des pH-Werts der Lösung von Schritt (b) auf etwa 9-12;
d. Filtrieren und Waschen des resultierenden Fällmittels von Schritt (c);
e. Kalzinieren des Fällmittels von Schritt (d) bei 300 °C bis 600 °C für 1 Stunde bis 6 Stunden, um die Additivzusammensetzung zu erhalten,

wobei das Verfahren ein Zugeben eines Oxidationsmittels zu dem Gemisch von Schritt (a) umfasst, optional wobei das Oxidationsmittel: (a) aus der Gruppe bestehend aus Wasserstoffperoxid, Chlorat, Perchlorat, Hypochlorit, Salpetersäure, Schwefelsäure, Kaliumpermanganat oder Gemischen davon ausgewählt ist; und/oder (b) in einer Menge von 100 % in einem Überschuss zu den Molen von Element D in der Additivzusammensetzung zugegeben wird.

4. Verfahren nach Anspruch 3, wobei die Salze von A, B, C und D aus der Gruppe bestehend aus Nitrat, Acetat, Chlorid und Sulfat ausgewählt sind.

5. Verfahren nach Anspruch 3 oder Anspruch 4, wobei das Verfahren ein Zugeben eines Tensids zu dem Gemisch von Schritt (a) umfasst, optional wobei das Tensid: (a) aus der Gruppe bestehend aus Polyvinylalkohol, 4-(1,1,3,3-Tetramethylbutyl)phenylpolyethylenglykol, Poloxamer 407, Polyethylenglykol, Natriumsalz von Polyacrylsäure oder Gemischen davon ausgewählt ist; und/oder (b) in einer Menge von 50 % in einem Überschuss zu den Molen von Element A, die in der Additivzusammensetzung vorliegen, zugegeben wird.

6. Verfahren nach einem der Ansprüche 3 bis 5, wobei das Fällungsmittel aus der Gruppe bestehend aus Natriumhydroxid, Natriumcarbonat, Oxalsäure, Natriumoxalat, Ammoniumoxalat oder Gemischen davon ausgewählt ist und in einer Menge zugegeben wird, um einen End-pH-Wert von mindestens 9 zu erhalten.

7. Verfahren nach Anspruch 3, wobei der pH-Wert der Lösung von Schritt (b) unter Verwendung von Tetramethylammoniumhydroxid justiert wird.

8. Verfahren nach einem der Ansprüche 3 bis 7, wobei das Fällmittel von Schritt (d) bei 500 °C für 4 Stunden kalziniert wird.

9. Katalysator, umfassend die Additivzusammensetzung nach Anspruch 1 oder Anspruch 2 und einen Mischmetalloxidkatalysator, optional wobei das Metall des Mischmetalloxidkatalysators aus der Gruppe bestehend aus Gruppe III und Gruppe IV des Periodensystems der Elemente ausgewählt ist.

10. Katalysator nach Anspruch 9, wobei der Mischmetalloxidkatalysator aus der Gruppe bestehend aus einem sulfatierten oder einem wolframierten Metalloxid von Zirkonium oder Aluminium oder Gemischen davon ausgewählt ist.

11. Katalysator nach Anspruch 9 oder Anspruch 10, wobei der Mischmetalloxidkatalysator ferner ein Gruppe-VIII-Metall umfasst, das aus der Gruppe bestehend aus Platin, Palladium, Rhenium, Rhodium, Iridium, Ruthenium, Gold oder Gemischen davon ausgewählt ist.

12. Katalysator nach einem der Ansprüche 9 bis 11, wobei die Additivzusammensetzung eine Fluoritphase und/oder eine Spinellphase und/oder eine Mullitphase aufweist, wenn sie mit Transmissionselektornenmikroskopie- oder Rastertransmissionselektronenmikroskopie-/energiedispersiven Röntgenanalyse-Techniken charakterisiert wird.

13. Methode zur Produktion eines Katalysators, umfassend das Verfahren nach einem der Ansprüche 3 bis 8, wobei die Additivzusammensetzung einem Mischmetalloxidkatalysator zugegeben wird, optional wobei etwa 0,5 bis 25 Gewichtsprozent der Additivzusammensetzung dem Mischmetalloxidkatalysator zugegeben werden, und ferner optional wobei die Methode einen Kalzinierungsschritt umfasst, nachdem die Additivzusammensetzung dem Mischmetalloxidkatalysator zugegeben wurde.

14. Verfahren zur Umwandlung von Kohlenwasserstoffen durch Inkontaktbringen einer Beschickung mit dem Katalysator nach einem der Ansprüche 9 bis 12, wobei das Kohlenwasserstoffumwandlungsverfahren aus der Gruppe bestehend aus Isomerisierung, Alkylierung oder Transalkylierung ausgewählt ist.

**Revendications**

1. Une composition d'additif comprenant un oxyde métallique mixte ayant la formule générale :

$$A_x B_y C_{(1-5)} D_z O_m |$$

dans laquelle :

A est le strontium ;
B est le cérium ou l'yttrium ;
C est le samarium ou l'ytterbium ;
D est le manganèse ou le ruthénium ;
x est un nombre défini par $0,5 < x < 4$
y est un nombre défini par $0 <= y <= l$
z est un nombre défini par $2 < z < 6$
m est un nombre qui rend la charge totale sensiblement neutre.

2. La composition d'additif selon la revendication 1, dans laquelle le pourcentage molaire de l'élément A est dans la plage de $0,1 < A < 0,25$ ; le pourcentage molaire des éléments B, C est dans la plage de $0,05 < B, C < 0,2$ ; le pourcentage molaire de l'élément D est dans la plage de $0,1 < D < 0,35$ ; et la charge de valence de l'oxygène est telle que la charge totale de la molécule est neutre.

3. Un procédé de préparation de la composition d'additif selon la revendication 1 comprenant :

    a. la dissolution des sels solubles dans l'eau de A, B, C et D pour obtenir la formule générale selon la revendication 1 ;
    b. l'ajout d'un agent de précipitation au mélange résultant de l'étape (a) ;
    c. l'ajustement du pH de la solution de l'étape (b) à environ 9 à 12 ;
    d. le filtrage et le lavage du précipité résultant de l'étape (c) ;
    e. la calcination du précipité de l'étape (d) à 300°C à 600°C pendant 1 heure à 6 heures pour obtenir la composition d'additif,

dans lequel le procédé comprend l'ajout d'un agent oxydant au mélange de l'étape (a), en option dans lequel l'agent oxydant est : (a) sélectionné dans le groupe constitué de peroxyde d'hydrogène, chlorate, perchlorate, hypochlorite, acide nitrique, acide sulfurique, permanganate de potassium ou des mélanges de ces derniers ; et/ou (b) ajouté dans une quantité de 100 % en plus des moles de l'élément D dans la composition d'additif.

4. Le procédé selon la revendication 3, dans lequel les sels de A, B, C et D sont sélectionnés dans le groupe constitué de nitrate, acétate, chlorure ou sulfate.

5. Le procédé selon la revendication 3 ou la revendication 4, dans lequel le procédé comprend l'ajout d'un tensioactif au mélange de l'étape (a), en option dans lequel ledit tensioactif est : (a) sélectionné dans le groupe constitué d'alcool polyvinylique, 4-(1,1,3,3, tétraméthylbutyl)phényl - polyéthylène glycol, poloxamère 407, polyéthylène glycol, sel de sodium d'acide polyacrylique ou des mélanges de ces derniers ; et/ou (b) ajouté dans une quantité de 50 % en plus des moles de l'élément A présent dans la composition d'additif.

6. Le procédé selon l'une quelconque des revendications 3 à 5, dans lequel l'agent de précipitation est sélectionné dans le groupe constitué d'hydroxyde de sodium, carbonate de sodium, acide oxalique, oxalate de sodium, oxalate d'ammonium ou des mélanges de ces derniers et ajouté dans une quantité pour obtenir une valeur pH finale d'au moins 9.

7. Le procédé selon la revendication 3, dans lequel le pH de la solution de l'étape (b) est ajusté à l'aide d'hydroxyde de tétraméthylammonium.

8. Le procédé selon l'une quelconque des revendications 3 à 7, dans lequel le précipité de l'étape (d) est calciné à 500°C pendant 4 heures.

9. Un catalyseur comprenant la composition d'additif selon la revendication 1 ou la revendication 2, et un catalyseur d'oxyde métallique mixte, en option dans lequel le métal du catalyseur d'oxyde métallique mixte est sélectionné dans le groupe constitué du Groupe III et du Groupe IV du tableau périodique.

10. Le catalyseur selon la revendication 9, dans lequel le catalyseur d'oxyde métallique mixte est sélectionné dans le groupe constitué d'un oxyde métallique de zirconium ou d'aluminium au sulfate ou au tungstène ou des mélanges de ces derniers.

11. Le catalyseur selon la revendication 9 ou la revendication 10, dans lequel ledit catalyseur d'oxyde métallique mixte comprend en outre un métal du Groupe VIII sélectionné dans le groupe constitué de platine, palladium, rhénium, rhodium, iridium, ruthénium, or ou des mélanges de ces derniers.

12. Le catalyseur selon l'une quelconque des revendications 9 à 11, dans lequel la composition d'additif a une phase fluorite et/ou une phase spinelle et/ou une phase mullite lorsque **caractérisé par** les techniques de la microscopie électronique en transmission ou la microscopie électronique en transmission à balayage / l'analyse à rayons X à dispersion d'énergie.

13. Un procédé de fabrication d'un catalyseur comprenant le procédé selon l'une quelconque des revendications 3 à 8, dans lequel la composition d'additif est ajoutée à un catalyseur d'oxyde métallique mixte, en option dans lequel environ 0,5 à 25 pour cent en poids de la composition d'additif est ajoutée au catalyseur d'oxyde métallique mixte, et en option en outre dans lequel le procédé comprend une étape de calcination après que la composition d'additif est ajoutée au catalyseur d'oxyde métallique mixte.

14. Un procédé de conversion d'hydrocarbures par le contact avec une alimentation du catalyseur selon l'une quelconque des revendications 9 à 12, dans lequel le procédé de conversion d'hydrocarbures est sélectionné dans le groupe constitué de l'isomérisation, l'alkylation ou la transalkylation.

Figure 1

**Figure 2**

**Figure 3**

**EP 3 294 451 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6107235 A
- US 6080904 A
- RU 2191627
- US 3032599 A
- US 3132110 A
- US 6448198 B
- EP 1002579 A
- RU 2171713
- US 7015175 B
- US 20140219878 A1
- US 20050161368 A1
- EP 1002579 A1
- US 9040762 B2
- EP 0532024 A1
- EP 0558148 A1
- US 6448198 B1
- EP 0532153 B1

### Non-patent literature cited in the description

- **HINO et al.** Synthesis of Solid superacid catalyst with acid strength of H0 < -16.04. *J.C.S. Chem. Comm.*, 1980, 851-852, 573
- Effect of isopropanol aging of Zr(OH)4 on n-hexane isomerization over Pt-SO42-/Al2O3-ZrO. *Catalysis Today*, October 2009, vol. 148 (1-2), 70-74
- Catalytic performance of Re/Ga2O3/WO3/ZrO2 catalyst for n-Hexane isomerization. *Chi.J. of. Catal.*, September 2009, vol. 30 (9), 859-863
- Catalytic performance of Re/Ga2O3/WO3/ZrO2 catalyst for n-Hexane isomerization. *Chi.J. of. Catal*, September 2009, vol. 30 (9), 859-863